(19) Europäisches Patentamt
European Patent Office
Office européen des brevets

(11) EP 2 432 759 B1

(12) EUROPÄISCHE PATENTSCHRIFT

(45) Veröffentlichungstag und Bekanntmachung des Hinweises auf die Patenterteilung:
**12.12.2012 Patentblatt 2012/50**

(21) Anmeldenummer: **10723028.6**

(22) Anmeldetag: **19.05.2010**

(51) Int Cl.:
***C07C 227/42*** (2006.01) ***C07C 229/24*** (2006.01)

(86) Internationale Anmeldenummer:
**PCT/EP2010/056856**

(87) Internationale Veröffentlichungsnummer:
**WO 2010/133618 (25.11.2010 Gazette 2010/47)**

# (54) VERFAHREN ZUR HERSTELLUNG EINES PULVERS ENTHALTEND EINES ODER MEHRERE DERIVATE DER GLYCIN-N,N-DIESSIGSÄURE UND/ODER EINES ODER MEHRERE DERIVATE DER GLUTAMIN-N,N-DIESSIGSÄURE UND METHYLGLYCIN-N,N-DIESSIGSÄURE-TRINATRIUMSALZPULVER

PROCESS FOR PREPARING A POWDER COMPRISING ONE OR MORE DERIVATIVES OF GLYCINE-N,N-DIACETIC ACID AND/OR ONE OR MORE DERIVATIVES OF GLUTAMINE-N,N-DIACETIC ACID AND METHYLGLYCINE-N,N-DIACETIC ACID TRISODIUM SALT POWDER

PROCÉDÉ DE PRÉPARATION D'UNE POUDRE CONTENANT UN OU PLUSIEURS DÉRIVÉS D'ACIDE GLYCINE-N,N-DIACÉTIQUE ET/OU UN OU PLUSIEURS DÉRIVÉS D'ACIDE GLUTAMINE-N, N-DIACÉTIQUE ET POUDRE DE SEL DE TRISODIUM DE L'ACIDE MÉTHYLGLYCINE-N,N-DIACÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **20.05.2009 EP 09160718**
**22.10.2009 US 253908 P**

(43) Veröffentlichungstag der Anmeldung:
**28.03.2012 Patentblatt 2012/13**

(73) Patentinhaber: **BASF SE**
**67056 Ludwigshafen (DE)**

(72) Erfinder:
- **MRZENA, Frank**
**67112 Mutterstadt (DE)**
- **KINDER, Hans-Juergen**
**67346 Speyer (DE)**
- **SCHOENHERR, Michael**
**67227 Frankenthal (DE)**
- **COX, Gerhard**
**67098 Bad Duerkheim (DE)**
- **SCHMIDT, Thomas**
**67433 Neustadt (DE)**
- **HUETT, Volker**
**68766 Hockenheim (DE)**

(56) Entgegenhaltungen:
**EP-A1- 1 334 961 EP-A2- 0 845 456**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 432 759 B1

**Beschreibung**

Die Erfindung betrifft ein Verfahren zur Herstellung eines Pulvers enthaltend eines oder mehrere Derivate der Glycin-N,N-diessigsäure und/oder eines oder mehrere Derivate der Glutamin-N,N-diessigsäure und Methylglycin-N,N-diessigsäure-Trinatriumsalz-pulver.

Derivate der Glycin-N,N-Diessigsäure haben komplexierende Eigenschaften für Erdalkali- und Schwermetallionen und werden in weiten Bereichen der Industrie zum Beispiel in der Wasch- und Reinigungsmittelindustrie oder bei der Behandlung von Metalloberflächen usw. eingesetzt. In vielen Anwendungen werden diese aktiven Komponenten als Feststoffe mit anderen Feststoffen zusammen als Mischungen, zum Beispiel in Tablettenform gebracht und als Geschirrspülerreinigungstabs eingesetzt. Die Herstellung der Pulver erfolgt dabei primär aus wässrigen Lösungen, was jedoch zu entsprechend aufwändigen und unwirtschaftlichen Massenkristallisationsverfahren (Verdampfungs- und Kühlungskristallisation) führt, weil die unsymmetrische Molekülform die Kristallisation stark_erschwert.

Daher werden diese Pulver großtechnisch meistens in Sprühtrocknungsanlagen hergestellt, was jedoch zu Feststoffen mit hohen amorphen Anteilen führt. Dies führt zu einem stark hygroskopischen Verhalten und schlechter Lager- und Weiterverarbeitbarkeit zum Beispiel in Tablettenpressen etc., was beispielsweise durch Nachbehandlung in Buildern für Waschmittel zur Zugabe von Benzoesäure kompensiert wird (vgl. US 3,932,316).

Die EP-A 08 45 456 beschreibt ein Verfahren zur Herstellung von Pulvern der obigen Komplexbildner mit erhöhtem Kristallinitätsgrad, wobei insbesondere von Ausgangsmassen mit Wasseranteilen im Bereich von 10-30% ausgegangen wird und bevorzugt Kristallisationskeime zugegeben werden. Dieses Verfahren führt zu überwiegend kristallinen Pulvern, erfordert aber wegen der zähen und pastösen Phasen während der Herstellung den Einsatz von aufwändigen Mischer-Kneter-Apparaten, um die Umwandlung in die kristallinen Modifikationen mit zu gewährleisten.

Es war demgegenüber Aufgabe der Erfindung, ein technisch einfacheres Verfahren zur Bereitstellung von Pulvern der obigen Komplexbildner mit erhöhtem Kristallinitätsgrad zur Verfügung zu stellen.

Die Lösung besteht in einem Verfahren zur Herstellung eines Pulvers enthaltend eines oder mehrere Derivate der Glycin-N,N-diessigsäure und/oder eines oder mehrere Derivate der Glutamin-N,N-diessigsäure mit einem Kristallinitätsgrad von ≥ 30%, ausgehend von einer wässrigen Lösung, enthaltend das eine oder die mehreren Derivate der Glycin-N,N-diessigsäure und/oder das eine oder die mehreren Derivate der Glutamin-N,N-diessigsäure, in einem Konzentrationsbereich von 20 bis 60%, bezogen auf das Gesamtgewicht der wässrigen Lösung, wobei

die wässrige Lösung in einem ersten Verfahrensschritt in einem Verdampfer mit rotierenden Einbauten, die in einem Abstand zur Innenwand des Verdampfers von ≤ 1% des Durchmessers des Verdampfers angeordnet sind, zu einem Kristallbrei mit einer Feststoffkonzentration im Bereich von 60 bis 85 Gew.-%, bezogen auf das Gesamtgewicht des Kristallbreis, aufkonzentriert wird, und wobei

der Kristallbrei in einem zweiten Verfahrensschritt in einem Pastenbunker und anschließend in einem Dünnschichtkontakttrockner ausreifen gelassen wird, wobei die Verweilzeit im Pastenbunker und im Dünnschichtkontakttrockner insgesamt ≥ 15 Minuten ist.

Das Verfahren geht aus von wässrigen Lösungen enthaltend eines oder mehrere Derivate der Glycin-N,N-diessigsäure und/oder eines oder mehrere Derivate der Glutamin-N,N-diessigsäure, bevorzugt eines oder mehrere Alkalisalze der Methylglycin-N,N-diessigsäure, im Folgenden abgekürzt mit MGDA bezeichnet, in einer Gesamtkonzentration im Bereich von etwa 20 bis 60%, bezogen auf das Gesamtgewicht der Lösung.

Bevorzugt werden Derivate der Glycin-N,N-diessigsäure oder der Glutamin-N,N-diessigsäure mit hoher Reinheit eingesetzt. Die Nebenprodukte aus der Synthese sollen in möglichst geringen Anteilen vorliegen, insbesondere soll der Anteil an 2-(Carboxymethyl-amino)-propionsäure-dinatriumsalz < 2%, Nitrilotriessigsäure-trinatriumsalz < 0,5%, Iminodiessigsäure-dinatriumsalz < 2% und Natriumhydroxid < 2% sein. Insbesondere wird von einer wässrigen Lösung ausgegangen, die das eine oder die mehreren Derivate der Glycin-N,N-diessigsäure und/oder der Glutamin-N,N-diessigsäure jeweils in einer Reinheit von ≥ 84%, bezogen auf die Trockenmasse, enthält.

Bevorzugt werden eines oder mehrere Derivate der Glycin-N,N-diessigsäure und/oder eines oder mehrere Derivate der Glutamin-N,N-diessigsäure eingesetzt, die hergestellt wurden, indem man entsprechende 2-Alkyl- bzw. 2-Alkenylglycine oder 2-Alkyl- bzw. 2-Alkenylglycinnitrile oder verdoppelte Glycine der Formel

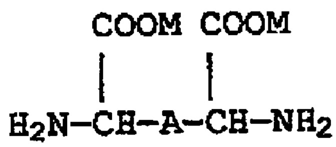

oder verdoppelte Glycinnitrile der Formel

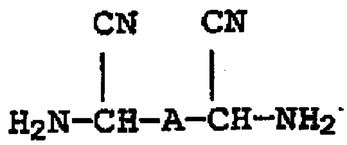

mit Formaldehyd und Cyanwasserstoff oder Alkalimetallcyanid oder Iminodiessigsäure oder Iminodiacetonitril mit entsprechenden Monoaldehyden oder Dialdehyden der Formel OHC-A-CHO und Cyanwasserstoff oder Alkalimetallcyanid umsetzt und anschließend noch vorhandene Nitrilgruppen zu Carboxylgruppen hydrolysiert

Die wässrige Lösung wird bevorzugt bei einer Temperatur im Bereich zwischen 20 und 90° eingesetzt.

Die wässrige Lösung enthaltend eines oder mehrere Derivate der Glycin-N,N-diessigsäure und/oder eines oder mehrere Derivate der Glutamin-N,N-diessigsäure wird in einem ersten Verfahrensschritt einem Verdampfer mit rotierenden Einbauten zugeführt und darin zu einem Kristallbrei mit einer Feststoffkonzentration im Bereich von 60 bis 85 Gew.-% aufkonzentriert.

Erfindungsgemäß überstreichen die rotierenden Einbauten die Innenwand des Verdampfers in einem sehr geringen Abstand von kleiner oder gleich 1 % des Durchmessers des Verdampfers. Durch den sehr geringen Abstand der rotierenden Einbauten zur Innenwand des Verdampfers wird eine hohe Scherrate im Flüssigkeitsfilm an der Innenwand des Verdampfers bewirkt. Dadurch wird eine intrinsische Kristallkeimbildung initiiert.

In einer bevorzugten Ausführungsform sind die rotierenden Einbauten dergestalt positioniert, dass sie an der Innenwand des Verdampfers kratzen.

Die Verdampfung in der ersten Verfahrensstufe erfolgt insbesondere in einem Temperaturbereich zwischen 50 und 140°C, vorzugsweise zwischen 80 und 110°C und in einem Druckbereich zwischen 0,1 bar absolut und 4 bar absolut, bevorzugt in einem Druckbereich zwischen 0,8 bar absolut und 1,2 bar absolut. Die erhöhte Temperatur in der ersten Verfahrensstufe wird insbesondere durch Beheizen der Wände des eingesetzten Verdampfungsapparats unter Ausbildung eines Doppelmantels, durch den ein Wärmeträger zirkuliert, gewährleistet.

Bevorzugt wird als Verdampfer im ersten Verfahrensschritt ein Sambay®-Verdampfer eingesetzt. Sambay®-Verdampfer sind spezielle Dünnschichtverdampfer, mit einem zentral angeordneten Kernrohr, auf dem bewegliche Wischerblätter angeordnet sind. Diese werden durch die Zentrifugalkraft an die beheizte Wand des Verdampfers gepresst. Durch Variation des Wischerblatttyps und somit des Anpressdruckes kann dieser Verdampfer an viele Problemstellungen optimal angepasst werden. Er ermöglicht bei niedriger Rotordrehzahl ein hohes Eindampfverhältnis bei gleichzeitig sehr geringen Ablaufmengen und ist v.a. für die Verarbeitung von belagbildenden Produkten geeignet. Der Sambay®-Verdampfer verarbeitet Viskositäten bis zu ca. 35.000 mPas.

Der nach dem ersten Verfahrensschritt resultierende Kristallbrei wird anschließend ausreifen gelassen, indem er geeigneten Apparaten zugeführt wird, die eine ausreichende Verweilzeit, von mindestens 15 Minuten, bevorzugt zwischen 15 Minuten und 1 Stunde, oder auch zwischen 15 Minuten und 3 Stunden, zur Verfügung stellen.

Hierzu wird der Kristallbrei aus dem ersten Verfahrensschritt zunächst einem Pastenbunker zugeführt, der bevorzugt mit Rührorganen zur Durchmischung des pastenförmigen Kristallbreis ausgestattet ist. Dem Pastenbunker kann zusätzlich eine Feinpulverfraktion, mit mittleren Partikeldurchmessern $\leq 200\ \mu m$ zugeführt und dem Kristallbrei zugemischt werden, bevorzugt in einem Anteil von bis zu 50%, bezogen auf das Gesamtgewicht der dem Pastenbunker zugeführten Stoffe. Dadurch können Feingutanteile, die im Gesamtverfahren anfallen, an dieser Stelle genutzt werden.

Der Kristallbrei mit ggf. zugemischtem Feinpulver wird anschließend einem Dünnschichtkontakttrockner zugeführt, worin bei einer Verweilzeit von etwa 0,5 bis 20 Minuten, insbesondere von etwa 10 Minuten und einer Temperatur im Bereich von etwa 60-140°C der Wassergehalt der Feststoffmischung dergestalt eingestellt wird, dass am Produktausgang aus dem Dünnschichtkontakttrockner ein Pulver erhalten wird, das überwiegend die Kristallmodifikation des Monohydrats oder des Dihydrats von MGDA aufweist.

Als Dünnschichtkontakttrockner werden beispielsweise schnelldrehende Schaufeltrockner unterschiedlicher Hersteller, beispielsweise Turbodryer der Fa. Vomm, horizontale Dünnschichttrockner der Fa. Buss, Kurzwegverdampfer der Fa. 3V Cogeim oder horizontale Zentrifugal-Trockner-Reaktoren der Fa. VRV, eingesetzt.

Das aus dem Dünnschichtkontakttrockner erhaltene Produkt zeichnet sich durch eine bessere Fließfähigkeit, geringere Hygroskopizität und bessere Lagerstabilität aus gegenüber Pulvern, die nach bekannten Trocknungsverfahren, beispielsweise durch Sprühtrocknung oder nach dem Mischer-Kneter-Verfahren, hergestellt wurden.

Gegenstand der Erfindung ist auch Methylglycin-N,N-diessigsäure-trinatriumsalz-Pulver mit einem Kristallinitätsgrad $\geq 30\%$ enthaltend eine erste kristalline Modifikation mit den nachfolgend angegebenen d-Werten in Angström bei den Beugungswinkeln 2-theta in °:

| 2-theta (°) | d Wert (Angström) |
|---|---|
| 8.4 | 10.5 |
| 9.5 | 9.3 |
| 11.1 | 8.0 |
| 13.2 | 6.7 |
| 13.9 | 6.35 |
| 15.8 | 5.6 |
| 16.5 | 5.36 |
| 16.84 | 5.26 |
| 17.34 | 5.11 |
| 17.67 | 5.02 |
| 18.92 | 4.69 |
| 20.29 | 4.37 |
| 21.71 | 4.09 |

(fortgesetzt)

| 2-theta (°) | d Wert (Angström) |
|---|---|
| 22.3 | 3.98 |
| 23.09 | 3.85 |
| 24.74 | 3.59 |
| 25.36 | 3.51 |
| 27.04 | 3.29 |
| 28.28 | 3.15 |
| 29.63 | 3.01 |
| 30.09 | 2.97 |

oder eine erste kristalline Modifikation entsprechend der obigen Definition und eine zweite kristalline Modifikation mit den d-Werten in Angström bei den jeweiligen Beugungswinkeln 2-theta in ° im Pulver-Diffraktogramm entsprechend der nachfolgenden Tabelle:

| 2-theta (°) | d Wert (Angström) |
|---|---|
| 8.2 | 10.80 |
| 10.5 | 8.40 |
| 15.55 | 5.70 |
| 16.47 | 5.38 |
| 17.09 | 5.18 |
| 18.10 | 4.90 |
| 18.82 | 4.71 |
| 21.00 | 4.23 |
| 21.35 | 4.16 |
| 22.64 | 3.92 |
| 23.69 | 3.75 |
| 24.73 | 3.60 |
| 26.75 | 3.33 |
| 28.93 | 3.08 |
| 29.88 | 2.99 |
| 31.46 | 2.84 |
| 31.88 | 2.80 |

Die Erfindung wird im Folgenden anhand von Ausführungsbeispielen sowie einer Zeichnung näher erläutert.
1 bar = $1.10^5$ Pa.

Ausführungsbeispiel 1 (zum Vergleich) Sprühtrocknung

Ein Mengenstrom von 60 kg/h einer wässrigen Lösung von MGDA mit einem Feststoffgehalt von 40% wurde in einem Plattenwärmetauscher-Verdampfer (Heizfläche 1,7 $m^2$) auf einen Feststoffgehalt von 59% eingedampft und in einem Abscheidebehälter abgeschieden. Die Eindampfung erfolgte bei einer Wandtemperatur von 152°C (Dampfbeheizung) und bei einem Druck von 2,5 bar abs im Abscheider.
Die eingedampfte Lösung wurde bei einer Temperatur von ca. 128°C mit einer Zahnradpumpe in die nachgeschaltete Kolbenmembranpumpe dosiert und mit einer Einstoffdüse in einem Sprühturm versprüht.
Der Sprühturm hatte einen Durchmesser von 800 mm und eine Länge von 12 m. Der Sprühturm wurde mit einer Luftmenge von 1400 kg/h und einer Gaseintrittstemperatur von 160°C betrieben. Die Produktaustrittstemperatur betrug 127°C und der Feststoffgehalt des Trockenproduktes 94,1%. Das Produkt wurde über einen 2-Punktaustrag (direkt am Sprühturm und am nachgeschalteten Filter) abgeschieden.
Das so hergestellte Produkt war ein rieselfähiges Pulver. Das Schüttgewicht betrug 529 kg/$m^3$. Die Röntgenstrukturanalyse zeigt, dass das Produkt amorph ist.
Das Lagerverhalten dieser Probe wurde im Exikkator-Test bewertet. Dazu wird eine Probe von 3 g in einem offenen Wägeschälchen in einen Exikkator bei 20°C und einer relativen Luftfeuchte von 76% über einen Zeitraum von 144 Stunden gelagert. Anschließend wird die Massezunahme der Probe ermittelt und die Rieselfähigkeit der Probe bewertet. Die Massezunahme betrug 27,1% und die Probe war angelöst, d.h. sie war nass und nicht mehr rieselfähig.

Ausführungsbeispiel 2 (zum Vergleich) Mischer-Kneter-Verfahren

Ein Mengenstrom von 20,5 kg/h einer wässrigen Lösung von MGDA mit einem Feststoffgehalt von 40% wurde in einem Plattenwärmetauscher (Heizfläche 1,7 $m^2$) auf eine Lösungstemperatur von 80°C vorgewärmt und mit einer Zahnradpumpe in einem CRP® 25 Conti-Kontakttrockner der Fa. List eindosiert.
Der List-Kontakttrockner ist ein Zweiwellenapparat mit den Innenabmessungen von 170*280 mm, einem Volumen von 31 Litern, einer Heizfläche von 1,3 $m^2$ und er wurde mittels Dampf auf eine Wandtemperatur von 174°C beheizt. Die Wellen wurden mit den Drehzahlen von 30 und 24 Umdrehungen pro Minute betrieben. In diesem Kontakttrockner wurde das Produkt auf einen Feststoffgehalt von 92% getrocknet.
Das so hergestellte Produkt war ein sehr gut rieselfähiges Granulat. Das Schüttgewicht betrug ca. 650 kg/$m^3$. Das Röntgenpulverdiffraktogramm zeigt, dass das Produkt amorphe und kristalline Anteile hat. Der Kristallinitätsgrad entsprechend oben beschriebener Analyse beträgt 30%.
Das Lagerverhalten der Probe wurde wie unter Beispiel 1 beschrieben ermittelt. Die Massenzunahme betrug 22,7% und die Probe war leicht verklumpt, d.h. sie war

nicht mehr rieselfähig, konnte aber durch leichtes Klopfen an das Wägeschälchen wieder in den rieselfähigen Zustand gebracht werden.

Ausführungsbeispiel 3 (zum Vergleich) Mischer-Kneter-Verfahren

Ein Mengenstrom von 32 kg/h einer wässrigen Lösung von MGDA mit einem Feststoffgehalt von 40% wurde in einem Plattenwärmetauscher-Eindampfer (Heizfläche 1,7 $m^2$) auf einen Feststoffgehalt von 61,8% eingedampft und mit einer Zahnradpumpe über eine Druckhalteventil in einen DTB® 25 Conti Kontakttrockner der Fa. List eindosiert. Die Eindampfung erfolgte bei einer Wandtemperatur von 142°C am Verdampfer und bei einem Druck von 2,5 bar abs. im Abscheidebehälter.
Der List-DTB 25 Conti-Kontakttrockner ist ein Einwellenapparat mit dem Innendurchmesser von 170 mm, einem Volumen von 30 Litern und einer Heizfläche von 1,2 $m^2$. Er wurde mittels Dampf auf eine Wandtemperatur von 186°C beheizt. Die Welle wurde mit der Drehzahl von 16 Umdrehungen pro Minute betrieben. In diesem Kontakttrockner wurde das Produkt auf einen Feststoffgehalt von 88,1% getrocknet.
Das so hergestellte Produkt war ein sehr gut rieselfähiges Granulat. Das Schüttgewicht betrug ca. 600 kg/$m^3$. Das Röntgenpulverdiffraktogramm zeigt, dass das Produkt amorphe und kristalline Anteile hat. Der Kristallinitätsgrad entsprechend oben beschriebener Analyse beträgt 27%.
Das Lagerverhalten der Probe wurde wie unter Beispiel 1 beschrieben ermittelt. Die Massenzunahme betrug 21,7% und die Probe war leicht verklumpt, d.h. sie war nicht mehr rieselfähig, konnte aber durch leichtes Klopfen an das Wägeschälchen wieder in den rieselfähigen Zustand gebracht werden.

Ausführungsbeispiel 4 (Erfindung)

Ein Mengenstrom von 3,3 kg/h einer wässrigen Lösung von MGDA mit einem Feststoffgehalt von 45,8% wurde in einem Labor-Sambay®-Verdampfer (Heizfläche 0,046 $m^2$) auf einen Feststoffgehalt von 65,9% eingedampft. Die Eindampfung erfolgte bei einer Wandtemperatur von 205°C bei Normaldruck.
Die eingedampfte Lösung wurde mit einer Temperatur von ca. 100°C in einem Dosierbunker von 8 Litern Inhalt aufgefangen und unter Rühren abgekühlt. Aus dem Dosierbunker wurde das Produkt mittels Dosierschnecke in einen schnelldrehenden Kontakttrockner gefördert.
Der Kontakttrockner hatte einen Durchmesser von 134 mm und eine Heizfläche von 0,166 $m^2$ und wurde mittels Dampf auf eine Wandtemperatur von 184°C beheizt. Er wurde mit einer Drehzahl von 276 Umdrehungen pro Minute betrieben. In diesem Kontakttrockner wurde das Produkt von Feststoffgehalt von 65,9% auf einen Feststoffgehalt von 91,6% getrocknet.
Das so hergestellte Produkt war ein gut rieselfähiges Granulat. Das Schüttgewicht betrug 548 kg/$m^3$. Das Röntgenpulverdiffraktogramm zeigt, dass das Produkt kristallin ist. Der Kristallinitätsgrad entsprechend oben beschriebener Analyse beträgt 39%.
Das Lagerverhalten der Probe wurde wie unter Beispiel 1 beschrieben ermittelt. Die Massenzunahme betrug 20,3% und die Probe war unverändert rieselfähig wie bei der Einwaage.

Ausführungsbeispiel 5 (Erfindung)

Ein Mengenstrom von 3,2 kg/h einer wässrigen Lösung von MGDA mit einem Feststoffgehalt von 45,5% wurde in einem Labor-Sambay®-Verdampfer (Heizfläche 0,046 $m^2$) auf einen Feststoffgehalt von ca. 69% eingedampft. Die Eindampfung erfolgte bei einer Wandtemperatur von 120°C bei reduziertem Druck von 0,5 bar.
Die eingedampfte Lösung wurde mit einer Temperatur von ca. 80°C in einem Dosierbunker von 8 Litern Inhalt aufgefangen und unter Rühren abgekühlt. Aus dem Dosierbunker wurde das Produkt mittels Dosierschnecke in einen schnelldrehenden Kontakttrockner gefördert.
Der Kontakttrockner hatte einen Durchmesser von 134 mm und eine Heizfläche von 0,166 $m^2$ und wurde mittels Dampf auf eine Wandtemperatur von 120°C beheizt. Er wurde mit einer Drehzahl von 275 Umdrehungen pro Minute betrieben. In diesem Kontakttrockner wurde das Produkt von Feststoffgehalt von 69% auf einen Feststoffgehalt von 88% getrocknet.
Das so hergestellte Produkt war ein gut rieselfähiges Granulat. Das Schüttgewicht betrug 555 kg/$m^3$. Das Röntgenpulverdiffraktogramm zeigt, dass das Produkt kristallin ist. Der Kristallinitätsgrad entsprechend oben beschriebener Analyse beträgt 58% der Modifikation 1.
Das Lagerverhalten der Probe wurde wie unter Beispiel 1 beschrieben ermittelt. Die Massenzunahme betrug 18% und die Probe war unverändert rieselfähig wie bei der Einwaage.

Die Figuren 1 bis 5 zeigen Röntgenpulver-Diffraktrogramme der nach den Ausführungsbeispielen 1 bis 5 erhaltenen Pulver und belegen die erhöhten Kristallinitätsgrade für nach dem erfindungsgemäßen Verfahren erhaltene Pulver (Figuren 4 und 5).

In den Figuren ist auf der Abszisse der Beugungswinkel 2-theta, in °, und auf der Ordinate die gemessene Intensität, in Counts (Impulse) (dimensionslos), aufgeführt.

Die Röntgenpulverdiffraktometer-Messungen wurden an einem Diffraktometer D8 Advance® der Fa. Bruker AXS (Karlsruhe) durchgeführt. Gemessen wurde in Reflexion mit Cu-K a-Strahlung mit einer variablen Blendeneinstellung primär- und sekundärseitig. Der Messbereich betrug 2° bis 80° 2-theta, die Schrittweite 0,01° und die Messzeit pro Winkelschritt 3,6 Sekunden.

Aus den Röntgenpulverdiffraktogrammen wurde der Kristallinitätsgrad in bekannter Weise ermittelt, indem, wie üblich, der Flächenanteil der kristallinen Phase und der amorphen Phase bestimmt wurde und hieraus

der Kristallinitätsgrad, KG, als das Verhältnis der Fläche der kristallinen Phase, $I_c$, zur Gesamtfläche, bestehend aus der Fläche der amorphen Phase, $I_a$, und der Fläche der kristallinen Phase, $I_c$, berechnet:

$$KG = I_c/(I_c+I_a).$$

Die Bestimmung des Kristallinitätsgrads kann insbesondere mit einem Softwareprogramm, beispielsweise dem Softwareprogramm TOPAS® der Fa. Bruker AXS durchgeführt werden.

Hierzu wird zunächst eine amorphe Probe vermessen und der Linienverlauf mit Hilfe von sechs Einzellinien in einem Profilfit angefittet. Danach werden die Linienlagen dieser Linien sowie ihre Halbwertsbreiten fixiert und diese Werte als "amorphe Phase" abgespeichert.

Bei der zu vermessenden Probe, für die der Kristallinitätsgrad bestimmt werden soll, wird nun der Flächenanteil der kristallinen Phase und der Flächenanteil der amorphen Phase bestimmt und hieraus nach der vorstehend angegebenen Formel der Kristallinitätsgrad KG berechnet.

Die amorphe Phase wird wie oben definiert verwendet.

Die kristalline Phase kann ebenfalls über ihre Einzellinienlagen analog zur amorphen Phase definiert werden, oder anhand folgender Gitterkonstanten, als sog. (hkl)-Phase (a = 33,63, b = 11,36 und c = 6,20 und Raumgruppe Pbcm), wobei die Gitterparameter frei verfeinerbare Variablen sind. Der Untergrund wird als Polynom 1. Grades angefittet.

Das Programm TOPAS® berechnet die optimale Anpassung zwischen gemessenem Diffraktogramm und dem theoretischen Diffraktogramm bestehend aus amorpher und kristalliner Phase.

## Patentansprüche

1. Verfahren zur Herstellung eines Pulvers enthaltend eines oder mehrere Derivate der Glycin-N,N-diessigsäure und/oder eines oder mehrere Derivate der Glutamin-N,N-diessigsäure mit einem Kristallinitätsgrad von ≥ 30%,
ausgehend von einer wässrigen Lösung, enthaltend das eine oder die mehreren Derivate der Glycin-N, N-diessigsäure und/oder das eine oder die mehreren Derivate der Glutamin-N,N'-diessigsäure, in einem Konzentrationsbereich von 20 bis 60 Gew.-%, bezogen auf das Gesamtgewicht der wässrigen Lösung, wobei die wässrige Lösung in einem ersten Verfahrensschritt einem Verdampfer mit rotierenden Einbauten, die in einem Abstand zur Innenwand des Verdampfers von ≤ 1% des Durchmessers des Verdampfers angeordnet sind, zu einem Kristallbrei mit einer Feststöffkonzentration im Bereich von 60 bis 85 Gew-%, bezogen auf das Gesamtgewicht des Kristallbreis, aufkonzentriert wird, und wobei der Kristallbrei in einem zweiten Verfahrensschritt in einem Pastenbunker und anschließend in einem Dünnschichtkontaktrockner ausreifen gelassen wird, und wobei die Verweilzeit im Pastenbunker und im Dünnschichtkontakttrockner insgesamt ≥ 15 Minuten ist.

2. Verfahren nach Anspruch 1, **dadurch gekennzeichnet, dass** die rotierenden Einbauten an der Innenwand des Verdampfers kratzen.

3. Verfahren nach Anspruch 1 oder 2, **dadurch gekennzeichnet, dass** als Derivat der Glycin-N,N-diessigsäure eines oder mehrere Alkalisalze der Methylglycin-N,N-diessigsäure eingesetzt werden.

4. Verfahren nach einem der Ansprüche 1 bis 3, dadurch gekennzeichhet, dass die wässrige Lösung das eine öder die mehreren Derivate der Glycin-N, N-diessigsäure und/oder der Glutamin-N,N=diessigsäure jeweils in einer Reinheit von ≥ 84%, bezogen auf die Trockenmasse, enthält.

5. Verfahren nach Ansprüch 4, **dadurch gekennzeichnet, dass** das eine oder die mehreren Derivate der Glycin-N,N-diessigsäure und/oder det Glutamin-N,N-digssigsaure hergestellt werden, indem man entsprechende 2-Alkyl- bzw. 2-Alkenylglycine oder 2-Alkyk- bzw. 2-Alkenylglycinnitrile oder verdoppelte Glycine der Förmel

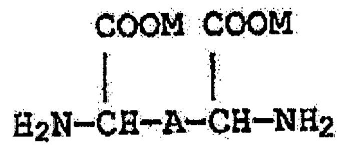

oder verdoppelte Glycinnitrile der Formel

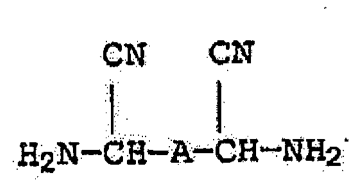

mit Formaldehyd und Cyanwasserstoff oder Alkalimetallcyanid oder Iminodiessigsäure öder Iminodiacetonitril mit entsprechenden Monoaldehyden oder Dialdehyden der Formel OHC-A-OHO und Cyanwasserstoff oder Alkalimetallcyanid
umsetzt und anschließend noch vorhandene Nitrilgruppen zu Carboxylgruppen hydrolysiert.

6. Verfahren nach einem der Ansprüche 1 bis 5, **da-**

**durch gekennzeichnet, dass** die wässrige Lösung enthaltend eines oder mehrere Derivate der Glycin-N,N-diessigsäure und/oder der Glutamin-N,N'-diessigsäure bei einer Temperatur im Bereich zwischen 20 und 90°C dem Verdampfer mit rotierenden Einbauten zugeführt wird.

**7.** Verfahren ach einem der Ansprüche 1 bis 6, **dadurch gekennzeichnet, dass** der Verdampfer mit rotierenden Einbauten ein Sambay®-Verdampfer ist.

**8.** Verfahren nach einem der Ansprüche 1 bis 7, **dadurch gekennzeichnet, dass** die Summe der Verweilzeiten im Pastenbunker und im Dünnschichtkontakttrockner insgesamt im Bereich zwischen 15 Minuten und 1 Stunde, bevorzugt im Bereich zwischen 15 Minuten und 3 Stunden liegt.

**9.** Verfahren nach einem der Ansprüche 1 bis 8, **dadurch gekennzeichnet, dass** der Verdampfer mit rotierenden Einbauten bei einer Temperatur im Bereich von 50-140°C, bevorzugt bei einer Temperatur im Bereich von 80-110°C, und bei einem Druck im Bereich von 0,1-4 bar absolut, vorzugsweise bei einem Druck vön 0,8-1,2 bar absolut, betrieben wird.

**10.** Verfahren nach einem der Anspruche 1 bis 9, **dadurch gekennzeichnet, dass** den Pastenbunker, zusätzlich zu dem Kristallbrei aus dem ersten Verfährensschritt bis zu 50 Gew.% Feihpülver mit einer mittleren Korngröße ≤ 200 μm, bezogen auf das Gesamtgewicht des Kristallbreis und des Feinpulvers zugegeben wird.

**11.** Verfahren nach einem der Ansprüche 1 bis 10, **dadurch gekennzeichnet, dass** der Dünnschichtkontakttrockner bei einer Temperatur im Bereich zwischen 60 und 140°C betrieben wird.

**12.** Methylglycin-N,N-diessigsäure-Trinatriumsalz-Pulver mit einem Kristallinitätsgrad ≤ 30%, enthaltend eine erste kristalline Modifikation mit den nachfolgend angegebenen d-Werten in Angström bei den Beugungswinkeln 2-theta in °:

| 2-theta (°) | d Wert (Angström) |
|---|---|
| 8.4 | 10.5 |
| 9.5 | 9.3 |
| 11.1 | 8.0 |
| 13.2 | 6.7 |
| 13.9 | 6.35 |
| 15.8 | 5.6 |
| 16.5 | 5.36 |
| 16.84 | 5.26 |
| 17.34 | 5.11 |
| 17.67 | 5.02 |
| 18.92 | 4.69 |
| 20.29 | 4.37 |
| 21.71 | 4.09 |
| 22.3 | 3.98 |
| 23.09 | 3.85 |
| 24.74 | 3.59 |
| 25.36 | 3.51 |
| 27.04 | 3.29 |
| 28.28 | 3.15 |
| 29.63 | 3.01 |
| 30.09 | 2.97 |

(fortgesetzt)

oder eine erste kristalline Modifikation entsprechend der obigen Definition und eine zweite kristalline Modifikation, entsprechend den d-Werten in Angström bei den jeweiligen Beugungswinkeln 2-theta in ° im Röntgen-Pulver-Diffraktogramm entsprechend der nachfolgenden Tabelle, jeweils gemessen mit Cu-K α-Strahlung mit einer variablen Blendeneinstellung primär- und sekundärseitig, in einem Messbereich von 2° bis 80°, mit einer Schrittweite von 0,01° und einer Messzeit pro Winkelschritt von 3,6 Sekunden, mit einem Diffraktometer D8 Advance® der Fa. Bruker AXS.

| **2-theta (°)** | **d Wert (Angström)** |
|---|---|
| 8.2 | 10.80 |
| 10.5 | 8.40 |
| 15.55 | 5.70 |
| 16.47 | 5.38 |
| 17.09 | 5.18 |
| 18.10 | 4.90 |
| 18.82 | 4.71 |
| 21.00 | 4.23 |
| 21.35 | 4.16 |
| 22.64 | 3.92 |
| 23.69 | 3.75 |
| 24.73 | 3.60 |

(fortgesetzt)

| 2-theta (°) | d Wert (Angström) |
|---|---|
| 26.75 | 3.33 |
| 28.93 | 3.08 |
| 29.88 | 2.99 |
| 31.46 | 2.84 |
| 31.88 | 2.80 |

**Claims**

1. A process for the preparation of a powder comprising one or more derivatives of glycine-N,N-diacetic acid and/or one or more derivatives of glutamine-N,N-diacetic acid with a degree of crystallinity of ≥ 30%, starting from an aqueous solution comprising the one or more derivatives of glycine-N,N-diacetic acid and/or the one or more derivatives of glutamine-N,N-diacetic acid in a concentration range from 20 to 60% by weight, based on the total weight of the aqueous solution, where the aqueous solution is concentrated in a first process step in an evaporator with rotating internals, which are arranged at a distance relative to the inside wall of the evaporator of ≤ 1% of the diameter of the evaporator, to give a crystal slurry with a solids concentration in the range from 60 to 85% by weight, based on the total weight of the crystal slurry, and where in a second process step the crystal slurry is left to ripen in a paste bunker and then in a thin-film contact dryer, and where the residence time in the paste bunker and in the thin-film contact dryer is in total ≥ 15 minutes.

2. The process according to claim 1, wherein the rotating internals scratch on the inside wall of the evaporator.

3. The process according to claim 1 or 2, wherein one or more alkali metal salts of methylglycine-N,N-diacetic acid are used as derivative of glycine-N,N-diacetic acid.

4. The process according to any one of claims 1 to 3, wherein the aqueous solution comprises the one or more derivatives of glycine-N,N-diacetic acid and/or of glutamine-N,N-diacetic acid in each case in a purity of ≥ 84%, based on the dry mass.

5. The process according to claim 4, wherein the one or more derivatives of glycine-N,N-diacetic acid and/or of glutamine-N,N-diacetic acid are prepared by reacting corresponding 2-alkyl- or 2-alkenylglycines or 2-alkyl- or 2-alkenylglycine nitriles or double glycines of the formula

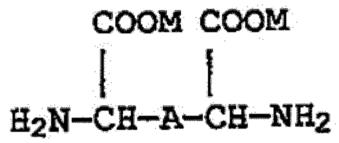

or double glycine nitrides of the formula

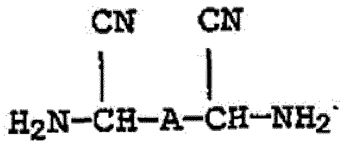

with formaldehyde and hydrogen cyanide or alkali metal cyanide or
iminodiacetic acid or iminodiacetonitrile with corresponding monoaldehydes or dialdehydes of the formula OHC-A-CHO and hydrogen cyanide or alkali metal cyanide
and then hydrolyzing any nitrile groups still present to give carboxyl groups.

6. The process according to any one of claims 1 to 5, wherein the aqueous solution comprising one or more derivatives of glycine-N,N-diacetic acid and/or of glutamine-N,N-diacetic acid is introduced into the evaporator with rotating internals at a temperature in the range between 20 and 90°C.

7. The process according to any one of claims 1 to 6, wherein the evaporator with rotating internals is a Sambay® evaporator.

8. The process according to any one of claims 1 to 7, wherein the sum of the residence times in the paste bunker and in the thin-film contact dryer is in total in the range between 15 minutes and 1 hour, preferably in the range between 15 minutes and 3 hours.

9. The process according to any one of claims 1 to 8, wherein the evaporator with rotating internals is operated at a temperature in the range from 50-140°C, preferably at a temperature in the range from 80-110°C, and at a pressure in the range from 0.1-4 bar absolute, preferably at a pressure of 0.8-1.2 bar absolute.

10. The process according to any one of claims 1 to 9, wherein, in addition to the crystal slurry from the first process step, up to 50% by weight of fine powder with an average particle size of ≤ 200 μm, based on the total weight of the crystal slurry and of the fine powder, is added to the paste bunker.

11. The process according to any one of claims 1 to 10, wherein the thin-film contact dryer is operated at a temperature in the range between 60 and 140°C.

**12.** A methylglycine-N,N-diacetic acid trisodium salt powder with a degree of crystallinity of ≥ 30%, comprising a first crystalline modification with the d values in angstroms given below at the diffraction angles 2-theta in °:

| 2-theta (°) | d value (angstroms) |
|---|---|
| 8.4 | 10.5 |
| 9.5 | 9.3 |
| 11.1 | 8.0 |
| 13.2 | 6.7 |
| 13.9 | 6.35 |
| 15.8 | 5.6 |
| 16.5 | 5.36 |
| 16.84 | 5.26 |
| 17.34 | 5.11 |
| 17.67 | 5.02 |
| 18.92 | 4.69 |
| 20.29 | 4.37 |
| 21.71 | 4.09 |
| 22.3 | 3.98 |
| 23.09 | 3.85 |
| 24.74 | 3.59 |
| 25.36 | 3.51 |
| 27.04 | 3.29 |
| 28.28 | 3.15 |
| 29.63 | 3.01 |
| 30.09 | 2.97 |

or a first crystalline modification corresponding with the above definition and a second crystalline modification corresponding with the d values in angstroms at the respective diffraction angles 2-theta in ° in the X-ray powder diffractogram corresponding to the table below, in each case measured with Cu-K $\alpha$-radiation with a variable diaphragm adjustment on the primary side and on the secondary side, in a measurement range of from 2° to 80° with a step width of 0.01° and a measurement time per angle step of 3.6 seconds, using a D8 Advance® diffractometer from Bruker AXS.

| 2-theta (°) | d value (angstroms) |
|---|---|
| 8.2 | 10.80 |
| 10.5 | 8.40 |
| 15.55 | 5.70 |
| 16.47 | 5.38 |
| 17.09 | 5.18 |
| 18.10 | 4.90 |
| 18.82 | 4.71 |
| 21.00 | 4.23 |
| 21.35 | 4.16 |
| 22.64 | 3.92 |
| 23.69 | 3.75 |
| 24.73 | 3.60 |
| 26.75 | 3.33 |
| 28.93 | 3.08 |
| 29.88 | 2.99 |
| 31.46 | 2.84 |
| 31.88 | 2.80 |

## Revendications

**1.** Procédé pour la préparation d'une poudre contenant un ou plusieurs dérivés de l'acide glycine-N,N-diacétique et/ou un ou plusieurs dérivés de l'acide glutamine-N,N-diacétique présentant un degré de cristallinité ≥ 30%, partant d'une solution aqueuse contenant ledit un ou lesdits plusieurs dérivés de l'acide glycine-N,N-diacétique et/ou ledit un ou lesdits plusieurs dérivés de l'acide glutamine-N,N-diacétique, dans une plage de concentration de 20 à 60% en poids, par rapport au poids total de la solution aqueuse, la solution aqueuse étant concentrée dans une première étape de procédé dans un évaporateur équipé d'encastrements rotatifs, qui sont disposés à une distance de la paroi interne de l'évaporateur ≤ 1% du diamètre de l'évaporateur, en une bouillie de cristaux présentant une concentration en solides dans la plage de 60 à 85% en poids, par rapport au poids total de la bouillie de cristaux, et la bouillie de cristaux étant portée à maturité dans une deuxième étape de procédé dans un conteneur à pâte puis dans un séchoir à couche mince par contact, le temps de séjour dans le conteneur à pâte et dans le séchoir à couche mince par contact étant au total ≥ 15 minutes.

**2.** Procédé selon la revendication 1, **caractérisé en ce que** les encastrements rotatifs raclent la paroi interne de l'évaporateur.

**3.** Procédé selon la revendication 1 ou 2, **caractérisé en ce qu'**on utilise, comme dérivé de l'acide glycine-N,N-diacétique, un ou plusieurs sels de métal alcalin de l'acide méthylglycine-N,N-diacétique.

**4.** Procédé selon l'une quelconque des revendications 1 à 3, **caractérisé en ce que** la solution aqueuse contient ledit un ou lesdits plusieurs dérivés, de l'acide glycine-N,N-diacétique et/ou de l'acide glutamine-N,N-diacétique à chaque fois en une pureté ≥ 84%, par rapport à la masse sèche.

**5.** Procédé selon la revendication 4, **caractérisé en ce que** ledit un ou lesdits plusieurs dérivés de l'acide glycine-N,N-diacétique et/ou de l'acide glutamine-N, N-diacétique sont préparés **en ce qu'**on transforme des 2-alkylglycines ou, selon le cas, des 2-alcénylglycines ou des 2-alkylglycinenitriles ou, selon le cas, des 2-alcénylglycinenitriles correspondants ou des doubles glycines de formule

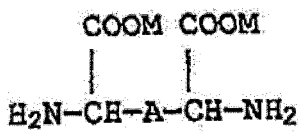

ou des doubles glycinenitriles de formule

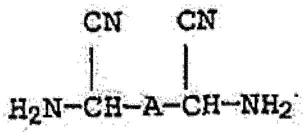

avec du formaldéhyde et de l'acide cyanhydrique ou du cyanure de métal alcalin ou de l'acide iminodiacétique ou de l'iminodiacétonitrile avec des monoaldéhydes ou des dialdéhydes correspondants de formule OHC-A-CHO et de l'acide cyanhydrique ou du cyanure de métal alcalin, puis on hydrolyse les groupes nitriles encore présents en groupes carbonyle.

**6.** Procédé selon l'une quelconque des revendications 1 à 5, **caractérisé en ce que** la solution aqueuse contenant un ou plusieurs dérivés de l'acide glycine-N,N-diacétique et/ou de l'acide glutamine-N/N-diacétique est introduite à une température dans la plage entre 20 et 90°C dans l'évaporateur équipé d'encastrements rotatifs.

**7.** Procédé selon l'une quelconque des revendications 1 à 6, **caractérisé en ce que** l'évaporateur équipé d'encastrements rotatifs est un évaporateur Sambay®.

**8.** Procédé selon l'une quelconque des revendications 1 à 7, **caractérisé en ce que** la somme des temps de séjour dans le conteneur à pâte et dans le séchoir à couche mince par contact est située au total dans la plage entre 15 minutes et 1 heure, de préférence dans la plage entre 15 minutes et 3 heures.

**9.** Procédé selon l'une quelconque des revendications 1 à 8, **caractérisé en ce que** l'évaporateur équipé d'encastrements rotatifs est exploité à une température dans la plage de 50-140°C, de préférence à une température dans la plage de 80-110°C, et à une pression dans la plage de 0,1-4 bars absolus, de préférence à une pression de 0,8-1,2 bar absolu.

**10.** Procédé selon l'une quelconque des revendications 1 à 9, **caractérisé en ce que** le conteneur à pâte est alimenté, en plus de la bouillie de cristaux de la première étape de procédé, en jusqu'à 50% en poids de poudre fine présentant une grosseur moyenne ≤ 200 $\mu$m, par rapport au poids total de la bouillie de cristaux et de la poudre fine.

**11.** Procédé selon l'une quelconque des revendications 1 à 10, **caractérisé en ce que** le séchoir à couche mince par contact est exploité à une température dans la plage entre 60 et 140°C.

**12.** Poudre de sel trisodique de l'acide méthylglycine-N, N-diacétique présentant un degré de cristallinité ≥ 30%, contenant une première modification cristalline présentant les valeurs d indiquées ci-dessus, en Angströms, aux angles de diffraction 2-thêta en ° :

| 2-thêta (°) | Valeur d (Angströms) |
|---|---|
| 8,4 | 10,5 |
| 9,5 | 9,3 |
| 11,1 | 8,0 |
| 13,2 | 6,7 |
| 13,9 | 6,35 |
| 15,8 | 5,6 |
| 16,5 | 5,36 |
| 16,84 | 5,26 |
| 17,34 | 5,11 |
| 17,67 | 5,02 |
| 18, 92 | 4,69 |
| 20,29 | 4,37 |
| 21,71 | 4,09 |
| 22,3 | 3,98 |
| 23,09 | 3,85 |
| 24,74 | 3,59 |
| 25,36 | 3,51 |

(suite)

| 2-thêta (°) | Valeur d (Angströms) |
|---|---|
| 27,04 | 3,29 |
| 28,28 | 3,15 |
| 29,63 | 3,01 |
| 30,09 | 2,97 |

ou une première modification cristalline correspondant à la définition ci-dessus et une deuxième modification cristalline correspondant aux valeurs d en Angströms aux différents angles de diffraction 2-thêta en ° dans le diffractogramme de rayons X sur des poudres selon le tableau ci-après, à chaque fois mesurées par un rayonnement Cu-K $\alpha$ avec un réglage variable du diaphragme, côté primaire et côté secondaire, dans une plage de mesure de 2° à 80°, avec une largeur de pas de 0,01° et un temps de mesure par pas angulaire de 3,6 secondes, à l'aide d'un diffractomètre D8 Advance® de la société Bruker AXS.

| 2-thêta (°) | Valeur d (Angströms) |
|---|---|
| 8,2 | 10,80 |
| 10,5 | 8,40 |
| 15,55 | 5,70 |
| 16,47 | 5,38 |
| 17,09 | 5,18 |
| 18,10 | 4,90 |
| 8,82 | 4,71 |
| 21,00 | 4,23 |
| 21,35 | 4,16 |
| 22,64 | 3,92 |
| 23,69 | 3,75 |
| 24,73 | 3,60 |
| 26,75 | 3,33 |
| 28,93 | 3,08 |
| 29,88 | 2,99 |
| 31,46 | 2,84 |
| 31,88 | 2,80 |

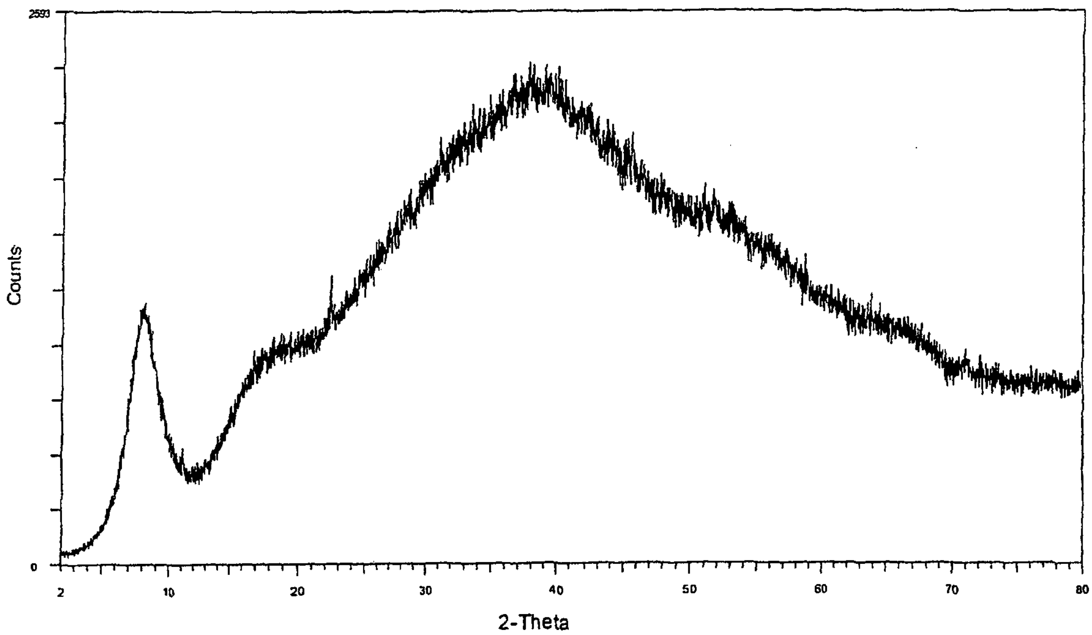
2593
Counts
0
2
10
20
30
40
50
60
70
80
2-Theta

Fig. 1

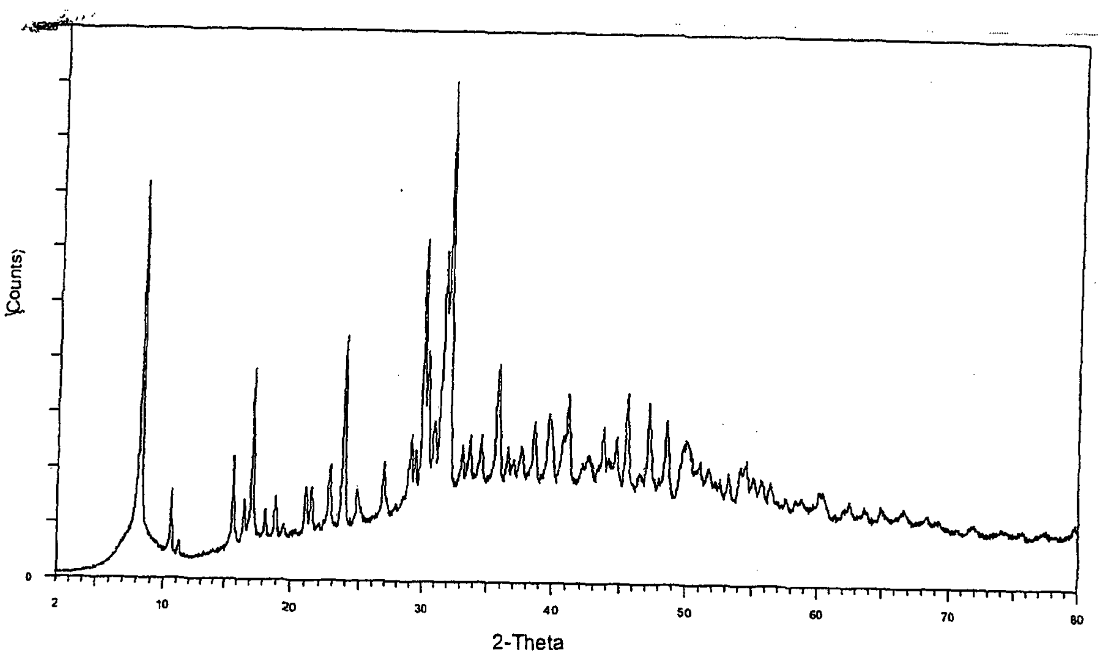
Counts
0
2
10
20
30
40
50
60
70
80
2-Theta

Fig. 2

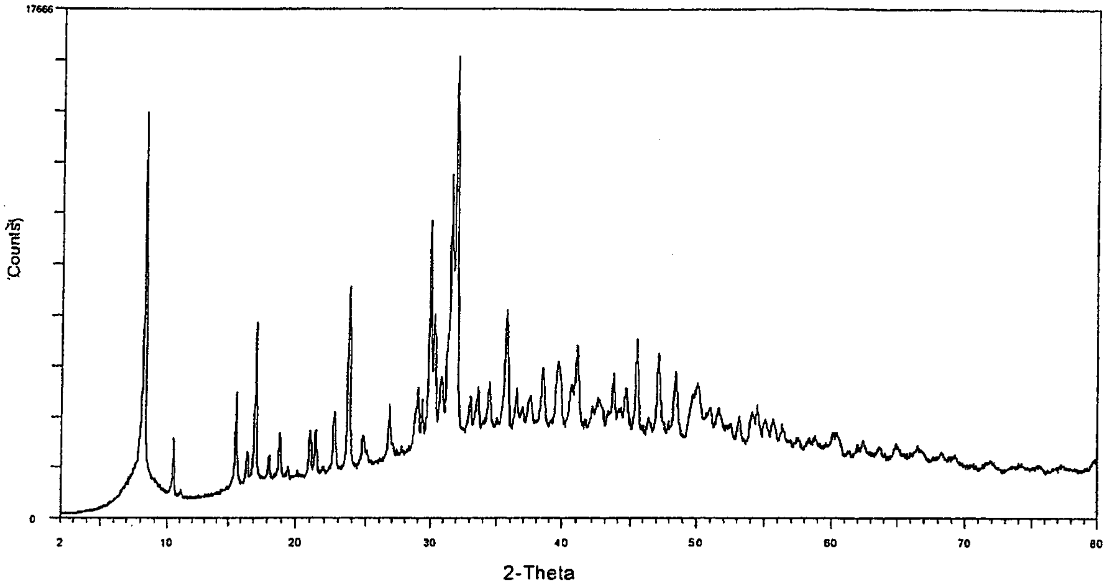
17666
0
Counts
2
10
20
30
40
50
60
70
80
2-Theta

Fig. 3

Fig. 4

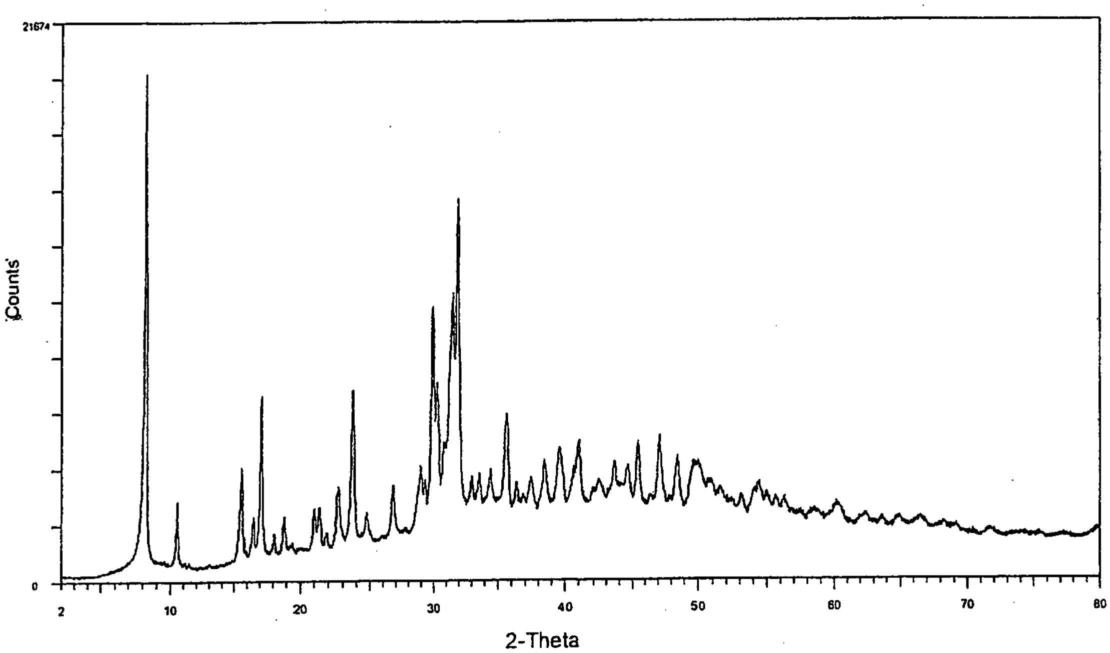
21674
0
Counts
2
10
20
30
40
50
60
70
80
2-Theta

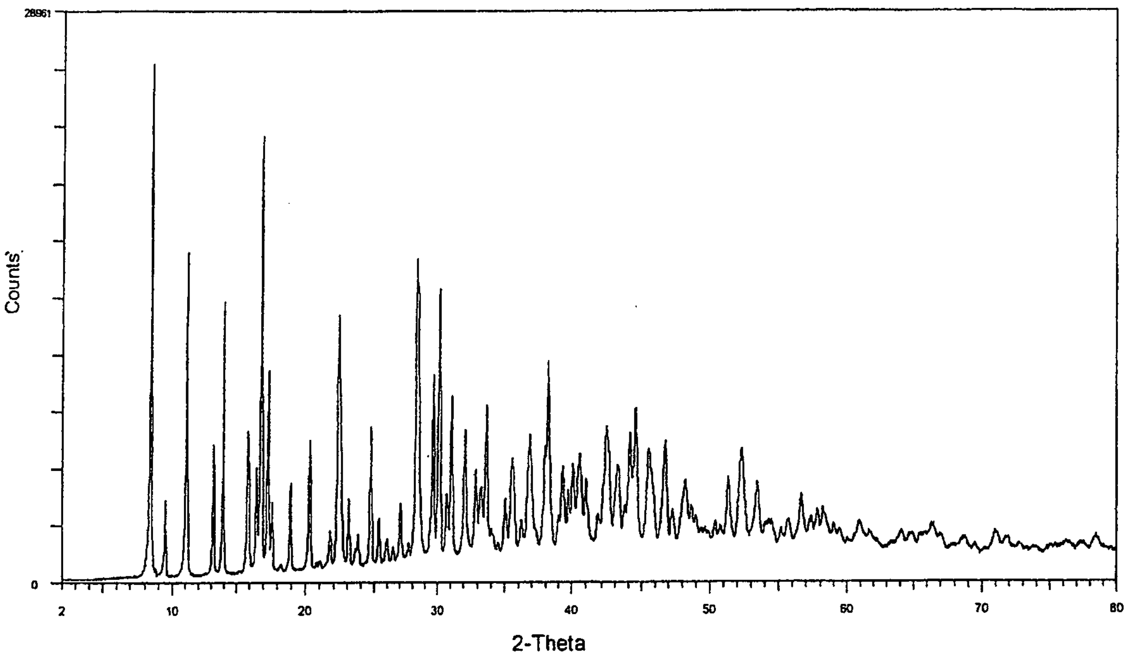


Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3932316 A
- EP 0845456 A